# EUROPEAN PATENT APPLICATION

(11) **EP 4 737 879 A1**
(43) Date of publication of application: **06.05.2026**
(21) Application number: 26160139.7
(22) Date of filing: 05.05.2021
(51) Int. Cl.: G01N 1/38, C12Q 1/04

(54) **METHOD FOR LIQUEFYING A RESPIRATORY SAMPLE AND FOR THE SUBSEQUENT DETECTION OF RESPIRATORY INFECTIONS IN SAID SAMPLE**

(30) Priority: 07.05.2020 ES 202030403
(62) Divisional of application: 21727193.1
(71) Applicant: Fundació Institut D'Investigació Sanitària Illes Balears, 07120 Palma de Mallorca (Islas Baleares) (ES); Servei de Salut de Les Illes Balears - Ibsalut, 07120 Palma de Mallorca (Islas Baleares) (ES)
(72) Inventor: DE LA RICA QUESADA, Roberto, 07120 Palma (ES); CLEMENTE XIMENIS, Antonio, 07120 Palma (ES); OLIVER PALOMO, Antonio, 07120 Palma (ES); ROJO MOLINERO, Estrella, 07120 Palma (ES)
(74) Representative: Hoffmann Eitle

(57) **Abstract**

The present invention relates to a method for liquefying respiratory samples, such as sputum samples. Samples of this type are characterized in that they can be highly viscous or semisolid, which means that to detect pathogenic microorganisms in them, they require prior treatment in order to make them more liquid and homogeneous. The liquefaction method proposed in the present innovation enables pathogenic microorganisms that cause respiratory infections to be subsequently detected.

## Description

The present invention relates to a method for liquefying respiratory samples, such as sputum samples. Samples of this type are characterized in that they can be highly viscous or semisolid, which means that to detect pathogenic microorganisms in them, they require prior treatment in order to make them more liquid and homogeneous. The liquefaction method proposed in the present innovation enables pathogenic microorganisms that cause respiratory infections to be subsequently detected.

Therefore, the present invention belongs to the area of medicine, and in particular, relates to the detection of respiratory tract infections.

### BACKGROUND OF THE INVENTION

Respiratory tract infections are the origin of a high percentage of cases of sepsis and they are particularly worrying in patients with chronic obstructive pulmonary disease (COPD) and cystic fibrosis. Among these infections, nosocomial infections such as those caused by the bacteria *Pseudomonas aeruginosa* must be detected as quickly as possible because they require an antibiotic treatment different from other pathogens due to their multidrug resistant character. Currently, this requires culturing the sputum of the patient, which may take several days. Therefore, even though bacteriological culture is the gold standard for bacteria detection, it is not suitable for guiding the first antibiotic regimen. In cases of sepsis or COPD exacerbations, administering inadequate antimicrobial therapy may result in grave consequences or even the patient's death. Moreover, it is not an adequate procedure for rapid detection at the point of care because it requires too much time and infrastructure.

Methods for pathogen detection in sputum samples are also known which require the extraction of nucleic acids, but again, this requires an additional time-consuming method that it is difficult to perform outside a specialised laboratory (Kukhtin A. V. et al. Anal. Chem. 2020, 92, 5311-5318).

In recent years, a new generation of biosensors has emerged as a rapid alternative to microbiological culture and nucleic acid extraction methods for detecting bacteria. In the context of detecting bacteria in sputum, such devices could reduce the time required to identify the pathogen, thus enabling the antibiotic treatment to be customised. However, the detection of bacteria and, in general, any pathogenic microorganism in sputum samples, or in another type of respiratory sample, is problematic because said samples are very viscous or semisolid and the pathogens may not be homogenously distributed within them.

The main component of respiratory samples are mucins, glycoproteins that oligomerise to generate a cross-linked scaffold where pathogenic microorganisms are trapped. Furthermore, in the case of some bacteria such as *Pseudomonas aeruginosa,* they are usually in the form of biofilms. This makes it even harder to detect them because, although the total concentration of bacteria in the sample may be high, they could be hidden inside the biofilm, and therefore they are undetectable. Due to this, a method that simultaneously liquefies the sample and disperses bacterial biofilms whenever they are present, in a few seconds and with simple procedure at the point of care, is required in order to rapidly detect pathogens in respiratory samples.

Nowadays, some methods are known for processing sputum samples for the subsequent detection of pathogenic microorganisms with a biosensor, which are detailed as follows. The use of thiolated compounds such as N-acetyl-cysteine (He, F. & Zhang, L. Anal. Sci. 18, 397-401 (2002); Kim, J. H. et al. Lab Chip 12, 1437-1440 (2012)) or a commercially available solution of dithiothreitol known as Sputasol (Massai, F. et al. Biosens. Bioelectron. 26, 3444-3449 (2011)) has been proposed for sputum liquefaction. These methods require incubation times of between 10 minutes and 1 hour and may require additional instruments such as a vortex mixer to disperse the sample or a thermostatic bath, which makes them harder to implement in field applications. Furthermore, these compounds are used for dispersing sputum, but they are not able to disperse the bacterial biofilm when said biofilm is present in the sample. Other methods based on the use of NaOH and surfactants suffer from the same issues (Inoue, S. et al. PLoS One 9, 1-7 (2014)). Finally, other methods have been proposed that use organic solvents such as chloroform (Buzid, A. et al. Sci. Rep. 6, 1-9 (2016)) or dichloromethane (Wen, K. Y. et al. ACS Synth. Biol. 6, 2293-2301 (2017)), which are flammable and can only be manipulated in a fume hood, making them inadequate for detecting pathogenic microorganisms at the point of care. Among the biosensors known for detecting microorganisms, one could cite, for example, those published in the following documents: Kumar N. et al. Analyst. 2018;143(2):359-373; Radhakrishnan R. et al. Biosensors. 2017;7(4):1-12; Cesewski E. et al. Biosensors and Bioelectronics. 2020, 159: 12214.

In view of the drawbacks existing in the current methods for processing respiratory samples for the subsequent detection of pathogenic microorganisms in the same, the present invention proposes an ultrafast, simple and economic processing method, such that it is possible to liquefy and homogenise the sample in a few seconds. Likewise, this makes it possible to detect pathogenic microorganisms in the sample quickly, accurately and efficiently, as well as allows it to be applied at points of care.

### DESCRIPTION OF THE INVENTION

The inventors have discovered that exclusively adding an aqueous solution of hydrogen peroxide (H₂O₂) to a sputum sample results in the liquefaction and homogenisation of said sample within approximately one minute.

The hydrogen peroxide decomposes into oxygen and water in the presence of the enzyme catalase found in the respiratory samples, as well as in the pathogenic microorganisms found in them, so that bubbles are generated. The generation of bubbles in the bulk of the sample favours the dispersion thereof until the total liquefaction and homogenisation thereof.

Therefore, in a first aspect, the present invention relates to a method for liquefying a respiratory sample, characterized in that it consists of adding an aqueous solution of hydrogen peroxide to said sample.

The method does not require any specific instruments (vortex mixer, thermostatic bath, etc.), or any other reagent or compound, or any additional step. In other words, adding the aqueous solution of hydrogen peroxide to the sample for approximately 1 minute is the only requirement for obtaining the liquefied and homogenised sample, ready to be used in the detection of any pathogenic microorganism within it.

In the present invention, the term "aqueous solution of hydrogen peroxide" refers to a solution comprising water (solvent) and hydrogen peroxide. More preferably, it refers to a solution containing water and hydrogen peroxide. In another preferred embodiment, it refers to a saline solution comprising hydrogen peroxide.

According to the present invention, "saline solution" is an aqueous solution containing at least one dissolved salt. As an example, the saline solution is the phosphate buffered saline (also known as PBS). This PBS solution is a 10 mM phosphate buffered solution, 0.14 M sodium chloride, 0.003 M potassium chloride at pH 7.4.

In a preferred embodiment of the invention, the aqueous solution has a concentration of hydrogen peroxide between 0.01 M and 1 M. More preferably, the concentration of hydrogen peroxide is 0.3 M.

In another preferred embodiment, the amount of the aqueous solution of hydrogen peroxide added to said sample is between 10 and 20 microliters per milligram of sample; more preferably, 20 microliters per milligram of sample.

According to the present invention, the term "respiratory sample" refers to an isolated biological sample from the respiratory tract of an individual. This sample comes from the upper respiratory tract, which comprises the nasal cavity, the paranasal sinuses, the pharynx and the larynx, or from the lower respiratory tract, which comprises the trachea, the primary bronchi and the lungs. They are viscous or semisolid samples. More preferably, the respiratory samples of the present invention are those comes from the lower respiratory tract.

In a preferred embodiment, the respiratory sample is a sputum, bronchial aspirate, bronchoalveolar lavage, nasopharyngeal swab, or bronchial brushing sample, or any other secretion generated in the respiratory tract.

The processing method described in this first aspect of the invention enables the liquefaction and homogenisation of the respiratory sample in a matter of seconds, such that it can be considered as an ultrafast method for pre-treating the same. Furthermore, it is advantageous because it is cheap and simple to perform, such that it is particularly useful for its application at the point of care. The resulting sample, already liquefied, can be directly used for the detection of pathogenic microorganisms therein, preferably by using a biosensor.

Another aspect of the invention refers to a method for detecting respiratory infections caused by pathogenic microorganisms in a respiratory sample, characterized by:
a) a first step for liquefying the respiratory sample according to the method defined in the first step of the invention and
b) a second step comprising the detection of the pathogenic microorganism in the liquefied respiratory sample obtained in the first step.

In the present invention, the term "pathogenic microorganism" is understood as a microorganism, such as a virus, bacterium or fungus, that causes disease or respiratory infection in humans or animals.

In a preferred embodiment, the pathogenic microorganism is a virus, bacterium or fungus that can infect the airways.

In another preferred embodiment, the pathogenic microorganism is a bacterium selected from the list comprising: *Pseudomonas aeruginosa, Streptococcus pneumoniae,* enterobacteria, *Staphylococcus aureus, methicillin-resistant Staphylococcus aureus, Klebsiella pneumoniae, Acinetobacter baumannii, Haemophilus influenzae or Legionella pneumophila.*

In another preferred embodiment, the pathogenic microorganism is a virus selected from the list comprising: coronavirus, influenza virus, rotavirus, cytomegalovirus and respiratory syncytial virus.

More preferably, the coronavirus is selected from: COVID-19, SARS-CoV-2, SARS and MERS and the influenza virus is selected from: the common flu virus (humans) and the avian influenza virus.

In another preferred embodiment, the pathogenic microorganism is a fungus selected from the list comprising: *Histoplasma capsulatum, Paracoccidioides brasiliensis, Blastomyces dermatitidis, Coccidioides immitis, Sporothrix schenckii, Candida spp., Aspergillus fumigatus, Torulopsis glabrata, Aspergillus spp., Pseudallescheria boydii, Cryptococcus neoformans,* and *Zygomycota* (such as *Rhizopus sp., Mucor sp.* and *Absidia sp.* etc).

The second step of detecting pathogenic microorganisms be performed using any known method that detects the pathogen itself or molecules of the same, such as enzymes, carbohydrates, nucleic acids, antigens and metabolites such as quorum sensing molecules. Preferably, the second step is performed using a biosensor, and preferably an immunosensor.

The term "biosensor" refers to any detection device made up of a transducer and a recognition element consisting of a biomolecule, for example, an enzyme, an aptamer, a nucleic acid, a carbohydrate or an antibody. In the case of an immunosensor, the biomolecule is an antibody. A wide variety of biosensors for detecting pathogenic microorganisms which can be used in the present invention in the detection step are known in the state of the art, as is known to a person skilled in the art.

Another aspect of the invention relates to a kit for detecting respiratory infections caused by pathogenic microorganisms in a respiratory sample, characterized in that it comprises or consists of an aqueous solution of hydrogen peroxide and a biosensor configured to detect the pathogenic microorganism under study.

In a preferred embodiment of the kit, the aqueous solution has a concentration of hydrogen peroxide between 0.01 M and 1 M, more preferably 0.3 M.

A final aspect of the invention relates to the use of the kit describe above for detecting respiratory infections in a respiratory sample.

Throughout the description and the claims, the word "comprises" and its variants are not intended to exclude other technical characteristics, additives, components or steps. On the other hand, the word "consists" is a closed expression, which cannot be interpreted as comprising products or methods that in turn include structural elements or steps of a method different from those disclosed in the claim. For those skilled in the art, other objects, advantages and features of the invention may be deduced from both the description and the practical use of the invention. The following examples and drawings are provided by way of illustration and are not meant to limit the present invention.

### BRIEF DESCRIPTION OF THE FIGURES

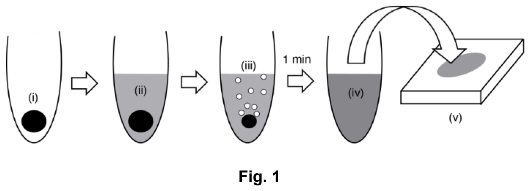

**Fig. 1** shows a schematic representation of the method for liquefying a respiratory sample such as sputum A hydrogen peroxide solution (ii) is added to the semi-solid sputum sample (i) so that the catalase present in the sample generates oxygen bubbles (iii) that disperse the sample. After 1 minute the sample has been liquefied (iv) and can be applied to a biosensor (v) for pathogen detection.

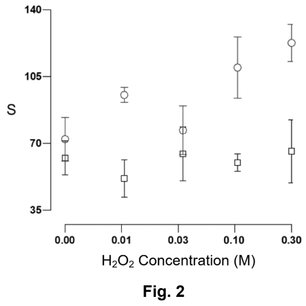

**Fig. 2** shows immunodetection of *Pseudomonas aeruginosa* after disaggregating sputum from a patient suffering an infection by *Pseudomonas aeruginosa* (red circle) or not suffering an infection (black square) with the proposed method. The colorimetric signal S increases when the concentration of peroxide increases because when more bubbles are generated more bacteria are released from the sputum infected by *Pseudomonas aeruginosa.* Error bars are the standard deviation of 3 experiments.

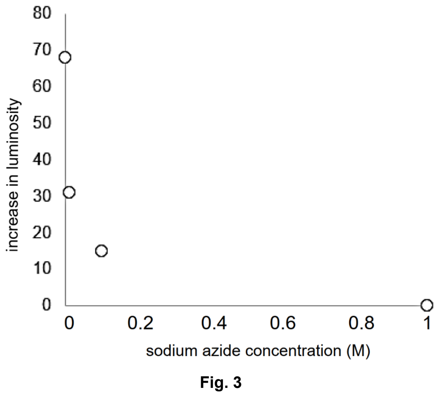

**Fig. 3** shows the increase in luminosity measured as the absolute value of the variation of pixel intensity from a sputum sample according to the experiment performed in example 3 to which an inhibitor of the enzyme catalase has been added.

### EXAMPLES

Next, the invention will be illustrated through experiments performed by the inventors, which shows the effectivity of the methods of the invention.

### Example 1: Liquefaction of a sputum sample

Firstly, solutions of hydrogen peroxide in PBS with a concentration of 0.01 M, 0.03 M, 0. 1 M, 0.3 M and 1 M were prepared.

200 microliters of each solution were added to 10 mg of sputum originating from a patient with a confirmed respiratory infection by *Pseudomonas aeruginosa.*

When adding the solution, the generation of bubbles was observed. The size and the number of bubbles increased with the concentration of peroxide in the solution. This was compared with the addition of water or PBS (without peroxide) which did not produce any bubbles, and therefore, did not liquefy the sputum sample. When the concentration of peroxide is 0.3 M, the sample liquefies in less than 1 minute.

The liquefied and homogenized samples obtained with this example were directly used to detect *P. aeruginosa* in them, as explained in Example 2.

### Example 2: Detection of P. aeruginosa in the liquified sputum samples obtained in example 1

The sputum samples that were previously liquefied as indicated in example 1 originated from a patient infected by *Pseudomonas aeruginosa* and from another patient with no infection.

*Pseudomonas aeruginosa* was detected in the liquefied sputum samples with paper immunosensors using gold nanoparticles as colorimetric probes. The method for manufacturing paper biosensors has been previously disclosed in several publications: Russell SM et al. ACS Sensors. 2017; 2:848-853; Russell SM et al. ACS Sensors. 2018;3(9):1712-1718; Alba-Patifio A. et al. Sensors Actuators, B Chem. 2018;273:951-954; Alba-Patiño A et al. ACS Sensors. 2020;5:147-153 and Alba-Patiño A. et al. Nanoscale Adv. 2020;2:253-1260. The method for manufacturing the biosensor used in this particular example has been previously disclosed in detail in the publication Alba-Patiño A. et al. ACS Sensors. 2020, 5:147-153.

To detect *Pseudomonas aeruginosa,* 10 µl of sputum (10 mg) liquefied with 200 µL of H₂O₂ at different concentrations between 0 and 0.3 M in PBS were deposited on the receiving surface of a 2x8 cm paper strip and let dry at room temperature (between 15 and 30 degrees Celsius) for 15 minutes. Subsequently, 1 mL of PBS supplemented with bovine serum albumin (5 mg/mL) was added to avoid the formation of non-specific interactions between nanoparticles and the paper substrate. Next, the paper was folded so that the reservoir containing nanoparticles decorated with an anti*-Pseudomonas aeruginosa* antibody comes in contact with the area containing the bacteria, pressing both surfaces with the aid of a clamp. After 5 minutes, excess nanoparticles were eliminated by washing the paper strip with PBS supplemented with Tween 20 (0.1%) and the colorimetric signal S generated by the nanoparticles on the receiving surface of the immunosensor, dependent on the presence of *Pseudomonas aeruginosa* in the applied sample, was measured with densitometry. To this end, the paper tests were scanned with a Brother MFC -1910 printer and the pixel intensity was calculated with the Histogram function of the ImageJ software. The signal S is taken as the absolute value of the number obtained after subtracting 255 from the pixel intensity value. The number 255 corresponds to the pixel intensity of pure white.

The results obtained are shown in Figure 2. In this figure, the sample with an infection by *Pseudomonas aeruginosa* that was treated only with PBS without hydrogen peroxide (0 M) yields a very low signal indistinguishable from the signal obtained with the sample without infection by *Pseudomonas aeruginosa.* As the concentration of hydrogen peroxide increases, the signal from the sample with infection by *Pseudomonas aeruginosa* increases because more antigens are released. The signal generated by the samples without *Pseudomonas aeruginosa* does not vary when the concentration of hydrogen peroxide increases, which demonstrates that the increase in signal observed in samples containing *Pseudomonas aeruginosa* is produced by the bacteria and not by the treatment with hydrogen peroxide. The highest signal was obtained with samples infected by *Pseudomonas aeruginosa* when the liquefaction step was performed with 0.3 M hydrogen peroxide.

### Example 3: Treatment of a sputum sample with hydrogen peroxide and a catalase inhibitor

10 mg of sputum were weighed out and 100 microliters of solutions containing PBS (phosphate buffer saline) with increasing concentrations of sodium azide (0, 0.01, 0.1 and 1 M), which is an inhibitor of the enzyme catalase, were added. The samples were incubated for 2 hours at room temperature. After pre-treatment, the azide was aspirated with a micropipette without touching the sputum samples. Subsequently 200 microliters of PBS with 0.3 M hydrogen peroxide were added for 1 minute. Photographs were taken before and after the treatment. In the sample without azide (0 M), the sputum was totally dissolved and the highest increase in luminosity was recorded after calculating the pixel intensity with image processing software such as ImageJ or Adobe Photoshop by selecting the area of interest and using the Histogram function. As the concentration of azide increased, the number of generated bubbles decreased and the sputum dissolved to a lesser extent. Therefore, the increase in luminosity decreased.

The results obtained in this experiment have demonstrated that the catalase activity in the samples is crucial for the generation of bubbles, and therefore, to dissolve or liquefy sputum, since when adding an inhibitor of this enzyme no dissolution or liquefaction of the sample is achieved.

## Claims

1. A method for liquefying a respiratory sample, **characterized in that** it consists of adding an aqueous solution of hydrogen peroxide to said sample.

2. The method, according to claim 1, wherein the aqueous solution has a concentration of hydrogen peroxide between 0.01 M and 1 M.

3. The method, according to claim 2, wherein the aqueous solution has a concentration of hydrogen peroxide of 0.3 M.

4. The method, according to any of the preceding claims 1 to 3, wherein the amount of the aqueous solution of hydrogen peroxide added to said sample is between 10 and 20 microliters per milligram of sample.

5. The method, according to any of the preceding claims 1 to 4, wherein the respiratory sample is a sputum sample, a sample of bronchial aspirate, bronchoalveolar lavage, nasopharyngeal swab or bronchial brushing.

6. A method for detecting respiratory infections caused by pathogenic microorganisms in a respiratory sample, **characterized by** comprising:
a) a first step for liquefying the respiratory sample according to the method defined in any of claims 1-5,
b) a second step comprising the detection of the pathogenic microorganism in the liquefied respiratory sample obtained in the first step.

7. The method, according to claim 6, wherein the pathogenic microorganisms are viruses, bacteria or fungi.

8. The method, according to claim 7, wherein the pathogenic microorganisms are bacteria selected from the list that comprises: *Pseudomonas aeruginosa, Streptococcus pneumoniae,* enterobacteria, *Staphylococcus aureus, methicillin-resistant Staphylococcus aureus, Klebsiella pneumoniae, Acinetobacter baumannii, Haemophilus influenzae or Legionella pneumophila.*

9. The method, according to claim 7, wherein the pathogenic microorganisms are viruses selected from the list that comprises coronavirus, influenza virus, rotavirus, cytomegalovirus and respiratory syncytial virus.

10. The method, according to claim 7, wherein the pathogenic microorganisms are fungi selected from the list that comprises: *Histoplasma capsulatum, Paracoccidioides brasiliensis, Blastomyces dermatitidis, Coccidioides immitis, Sporothrix schenckii, Candida spp., Aspergillus fumigatus, Torulopsis glabrata, Aspergillus spp., Pseudallescheria boydii, Cryptococcus neoformans,* and *Zygomycota.*

11. The method, according to any of the preceding claims 1 to 9, wherein the detection of the pathogenic microorganism in the second step is performed with a biosensor.

12. A kit for detecting respiratory infections caused by pathogenic microorganisms in a respiratory sample, **characterized in that** it comprises or consists of an aqueous solution of hydrogen peroxide and a biosensor configured to detect the pathogenic microorganism under study.

13. The kit, according to claim 12, wherein the aqueous solution has a concentration of hydrogen peroxide between 0.01 M and 1 M.

14. Use of the kit described in any of claims 12 or 13 for detecting respiratory infections in a respiratory sample.
